# EUROPEAN PATENT SPECIFICATION

(11) **EP 2 900 084 B1**
(45) Date of publication and mention of the grant of the patent: **04.04.2018**
(21) Application number: 14829343.4
(22) Date of filing: 25.02.2014
(51) Int. Cl.: A23J 3/34, A23L 23/00, A23L 27/23, A23L 27/26, C12P 13/14, C12P 19/32, A23L 27/24

(54) **METHOD FOR PREPARING NATURAL BEEF FLAVOR**
VERFAHREN ZUR HERSTELLUNG VON NATÜRLICHEM RINDFLEISCHAROMA
PROCÉDÉ DE PRÉPARATION D'ARÔME NATUREL DE BOEUF

(30) Priority: 23.07.2013 KR 20130086953
(43) Date of publication of application: 05.08.2015
(73) Proprietor: Cj Cheiljedang Corporation, Seoul 100-400 (KR)
(72) Inventor: LEE, Sung Hun, Goyang-si Gyeonggi-do 411-741 (KR); EOM, So Youn, Seoul 152-774 (KR); PARK, Jae Seung, Suwon-si Gyeonggi-do 441-807 (KR); OH, Eun Seon, Seongnam-si Gyeonggi-do 463-970 (KR); LEE, Kwang Hee, Seongnam-si Gyeonggi-do 462-835 (KR); JANG, Suk Min, Seoul 152-857 (KR); KANG, Dae Ik, Goyang-si Gyeonggi-do 411-750 (KR); CHUNG, Won Dae, Seoul 150-894 (KR)
(74) Representative: Regimbeau
(86) International application number: PCT/KR2014/001488
(87) International publication number: WO 2015/012464

(56) References cited:
- WO-A1-02/051984
- WO-A1-2010/108901
- WO-A2-2005/023850
- JP-A- 2000 000 088
- KR-A- 20000 002 408
- KR-A- 20090 078 585
- KR-A- 20090 078 585
- KR-A- 20120 076 841
- US-A- 3 385 761
- US-A- 5 147 667
- US-A- 5 164 306
- US-A1- 2001 003 593
- US-A1- 2004 166 200
- US-A1- 2012 178 125
- US-A1- 2012 315 354
- US-B2- 7 244 608

## Description

### Technical Field

The present invention relates to a method for preparing a natural beef flavor, and more particularly to a method of preparing a natural beef flavor using an inosine-5'-monophosphate (IMP) fermented broth or a glutamic acid fermented broth prepared by a two-step fermentation process comprising a first fermentation step for fungal fermentation and a second fermentation step for bacterial fermentation.

### Background Art

Amino acids and peptides are used as flavor components. In recent years, natural flavoring materials including amino acids and peptides extracted by fermenting vegetable protein sources, such as soybeans, wheat or corn, with microorganisms, such as fungi, Bacillus, lactic acid bacteria or yeast, and hydrolyzing the fermentation product, have been developed in various ways.

Generally, technology has been developed to improve the extracted taste components (amino acids and peptides) by increasing the degree of hydrolysis of the vegetable protein sources or increase price competitiveness by increasing the yield in terms of mass. However, when only vegetable protein sources are used, there is a disadvantage in that no nucleic acid component is present in the resulting flavoring material, and thus the umami taste intensity of the flavoring material is weak. In addition, flavoring materials are required to contain glutamic acid together with nucleic acids such as inosine monophosphate (IMP) or guanosine monophosphate (GMP) in order to increase the commercial value thereof.

In recent years, in order to overcome the above-described shortcoming of the vegetable protein hydrolysate, natural nucleic acid-containing materials such as yeast extracts have generally been used. However, the yeast extracts may adversely affect the inherent flavor of processed foods due to an off-flavor and off-odor resulting from the peculiar fermented odor thereof, and the nucleic acid content thereof is limited (maximum nucleic acid content: 20%). In addition, mixtures of vegetable protein materials with yeast extracts for use in natural processed foods have low price competitiveness compared to conventional flavoring materials such as monosodium glutamate (MSG), nucleic acid IG, or hydrolyzed vegetable protein (HVP).

Meanwhile, IMP together with GMP is a substance that is widely used as a food flavoring additive. When IMP is used specifically with monosodium glutamate (MSG), it has a significant ability to improve taste. Thus, IMP is a nucleic acid-based flavoring substance that is receiving attention as a flavoring substance.

Methods for fermenting IMP and GMP for use as flavoring substances include a method of degrading yeast RNA, and a method of preparing IMP and GMP by two steps (fermentation and chemical phosphorylation) after preparation of inosine and guanosine. US2012315354 discloses a process for the preparation of a taste enhancing savoury base comprising naturally derived compounds taken in the group consisting of glutamate, IMP and GMP, comprising fermenting a substrate using a microorganism of genus taken in the group consisting of Corynebacterium glutamicum, Corynebacterium ammoniagenes, Brevibacterium lactofermentum, Corynebacterium casei, Corynebacterium efficiens and Bacillus subtilis, wherein the substrate is obtained by the enzymatic hydrolysis of a plant material or by the enzymatic hydrolysis of starch. However, in recent years, in various countries, including Europe, standards for natural flavors have been strengthened and regulations have become more severe, and thus flavors which are not made of only natural components but are prepared by performing chemical processes or adding additional components are not recognized as natural flavors. For this reason, natural nucleic acid components should be prepared using a method directly fermenting sugar with bacteria.

Under such circumstances, the present inventors have made extensive efforts to prepare a natural beef flavor containing no additional component without performing any chemical process, and as a result, have found that, when an IMP or glutamic acid fermented broth produced by a two-step fermentation process comprising a first fermentation step for fungal fermentation and a second fermentation step for bacterial fermentation is used, a variety of effective flavors can be produced even when only the fermented broth is used for a subsequent reaction, thereby completing the present invention.

### Disclosure of Invention

### Technical Problem

It is an object of the present invention to provide a method of preparing a natural beef flavor containing no additional components, without performing any chemical process, and using an inosine-5'-monophosphate (IMP) fermented broth or a glutamic acid fermented broth prepared by a first fermentation step for fungal fermentation and a second fermentation step for bacterial fermentation.

### Solution to Problem

The invention is defined by the appended set of claims. To achieve the above objects, in one aspect, the present invention provides a method for preparing a natural beef flavor, the method comprising the steps of:
(a) fermenting a vegetable protein source with Aspergillus sp. microorganism to obtain a grain fermented broth; (b) fermenting a part of the grain fermented broth with Corynebacterium sp. or Brevibacterium sp. microorganism to prepare an inosine-5'-monophosphate (IMP) fermented broth; and (c) mixing another part of the grain fermented broth of step (a) and the IMP fermented broth of step (b), and reacting the mixture at 80 to 120°C at 0.8 to 1.5 bar for 0.5 to 24 hours.

The method may further comprise in step b) fermenting another part of the grain fermented broth with Corynebacterium sp. or Brevibacterium sp. microorganism to prepare a glutamic acid fermented broth, and in step (c) further mixing the glutamic acid fermented broth with the mixture obtained from step (c).

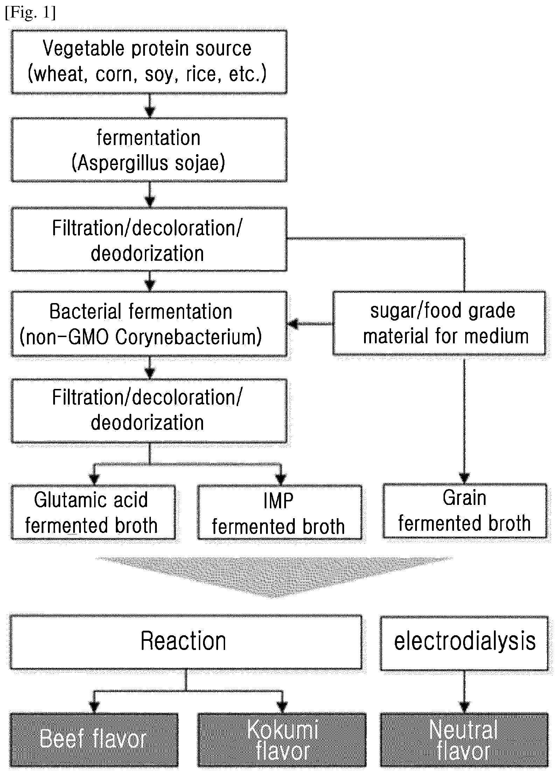

FIG. 1 schematically shows a method for preparing a natural flavor according to the present invention.

A glutamic acid fermented broth, an IMP fermented broth and a grain fermented broth are prepared by a first fermentation step for fungal fermentation and a second fermentation step for bacterial fermentation, and are subjected to a third step wherein a reaction or treatment suitable for each flavor, thereby preparing each natural flavor.

Thus, the present invention is characterized in that a natural IMP fermented broth or a glutamic acid fermented broth that are used as raw materials for preparing natural flavors are prepared by a two-step fermentation process comprising a first fermentation step for fungal fermentation and a second fermentation step for bacterial fermentation.

The first fermentation step for fungal fermentation is a step of producing peptides and amino acids using a protein source. When only the first fermentation step for fungal fermentation is carried out, large amounts of peptides and amino acids can be produced, and thus glutamic acid that may be used as a flavor can be produced. However, there is a disadvantage in that a nucleic acid substance, such as IMP or guanosine monophosphate (GMP), which can give an umami taste, cannot be produced. In addition, there is a problem in that, when a vegetable protein source is used, it is subjected only to a proteolytic process, and thus the peptide and amino acid that can be produced depend on the concentration or content of protein in the protein source. For example, when bean is used, the content of glutamic acid in the produced fermented broth will be less than 10%, and when wheat gluten is used, the content of glutamic acid in the produced fermented broth will be less than 15%.

Meanwhile, the second fermentation step for bacterial fermentation is a step of preparing nucleic acid and glutamic acid fermented broths. When only the second fermentation step for bacterial fermentation is carried out, nucleic acid and glutamic acid can be effectively produced, but there is a shortcoming in that the content of other amino acids and peptides is produced in a concentration of less than 1%, and thus the fermented broth with bacteria cannot be used as a flavor, even though it has a good umami taste. In other words, there is a shortcoming in that, in order to use the fermented broth, obtained only by bacterial fermentation, as a flavor, additional components are required to be added to the fermented broth so that the fermented broth can be applied to foods.

Accordingly, the present inventors have developed a method of preparing a natural beef flavor, while overcoming the shortcoming of the fungal fermentation process and the bacterial fermentation process, by using only the prepared fermented broth without adding any additional component, and without carrying out any chemical process.

To prepare a nucleic acid and glutamic acid fermented broth using the two-step fermentation process, various mineral salts, amino acids and vitamins together with a carbon source and a nitrogen source are required. Particularly, in the prior art, yeast extract or hydrolyzed vegetable protein (HVP) was used as the nitrogen source, but in this case, there were shortcomings in that the resulting fermented broth had an off-flavor and an off-odor and in that the yield is somewhat low. In addition, the content of flavoring components in the resulting culture broth, as well as the overall flavor, greatly differs depending on various materials that are used for bacterial fermentation. Thus, in the present invention, a grain fermented broth (grain protein hydrolysate), which is obtained by fungal fermentation (first fermentation step) and can contain various amino acids and peptides while serving as a nitrogen source, is used as a substrate for the second fermentation step for bacterial fermentation.

In conventional processes for preparing nucleic acid or MSG using bacteria, special importance was attached to increasing only the concentrations of nucleic acid and MSG in culture broths and the yields thereof. This increase was effective in increasing the umami taste, but in some cases, it adversely affected the flavor of the resulting flavoring substances. However, the various natural flavors prepared by using the 3 steps of the present invention have an advantage in that the flavor can be improved by controlling the concentrations of IMP and glutamic acid, which are umami taste components, as well as various amino acids, saccharides, organic acids and inorganic ions, etc.

Herein, each step of the inventive method for preparing a natural beef flavor will be described.

Step (a) of the method of the present invention is a step of fermenting a vegetable protein source with fungi to obtain a grain fermented broth.

In step (a), fungal fermentation is performed using a vegetable protein source, thereby obtaining a grain fermented broth that contains various amino acids and peptides and includes components such as glutamine, which can be used as a nitrogen source in the second fermentation step for bacterial fermentation. Specifically, fungi are cultured using a grain material as a substrate to prepare a protease-containing cell culture broth, which is then added to a vegetable protein source, followed by hydrolysis, thereby preparing a grain protein hydrolysate. Then, the grain protein hydrolysate is filtered, and cells are removed therefrom, thereby preparing a grain fermented broth. The grain protein hydrolysate may have a total nitrogen content of 2% (w/v) or more in order to provide a nitrogen source for the second fermentation step for bacterial fermentation.

As the vegetable protein source, any material known in the art may be used in the present invention, as long as it can be fermented with fungi. Examples of the vegetable protein source include, but are not limited to, soybean, corn, rice, wheat gluten, etc. Meanwhile, when wheat gluten is used, it can advantageously increase the yield of bacterial fermentation, because it contains a large amount of glutamine. Thus, wheat gluten may preferably be used as the vegetable protein source.

The fungi that are used in the present invention are *Aspergillus* sp. microorganisms, and more preferably *Aspergillus orizae* or *Aspergillus sojae* microorganisms, but are not limited thereto.

In an example of the present invention, the first fermentation step for fungal fermentation was carried out using *Aspergillus sojae* CJCC_080124P (KCCM11026P) as described in Korean Patent Registration No.10-1191010 (corresponding to PCT International Publication No.WO2011-046249).

As used herein, the term "grain fermented broth" refers to a product obtained by fermenting a vegetable protein source (grain) with fungi. The grain fermented broth may be used as a substrate for the second fermentation step for bacterial fermentation and may also be used in a final step of preparing a flavor by subjecting the grain fermented broth to filtration and cell removal processes after fermentation. Thus, the grain fermented broth may be used for two purposes as described above.

Step (b) is a step of fermenting the grain fermented broth, obtained in step (a), with bacteria to prepare an IMP fermented broth. In step (b) may further comprise a step of fermenting the grain fermented broth with bacteria to prepare a glutamic acid fermented broth. Specifically, the grain fermented broth obtained in the first fermentation step for fungal fermentation is used as a substrate for bacterial fermentation, and the IMP fermented broth and/or the glutamic acid fermented broth may be prepared by subjecting the grain fermented broth to bacterial fermentation in a medium supplemented with a carbon source.

The bacterial fermentation can be performed by a general bacterial culture method known in the art, and preferably, it may be composed of three steps: flask culture, scalp-up culture, and main culture. Specifically, flask culture and expansion culture are performed using primary culture medium and secondary culture medium in order to achieve scale-up, after which bacterial fermentation is performed using the grain fermented broth of step (a) as a substrate in main culture medium while continuously supplying additional sugar, thereby obtaining an IMP fermented broth and a glutamic acid fermented broth.

The medium for the bacterial fermentation may comprise a carbon source such as glucose, fructose or the like. Particularly, the medium for preparing the glutamic acid fermented broth may comprise raw sugar as a carbon source. The medium may comprise various mineral salts, vitamins, amino acids and the like, and the composition of the medium may vary depending on the desired fermentation product that is the IMP fermented broth or the glutamic acid fermented broth.

For example, when the IMP fermented broth is to be prepared, the main culture medium may comprise glucose, fructose, magnesium sulfate, phosphoric acid, potassium hydroxide and the grain fermented broth. Preferably, the main culture medium may comprise, based on the total volume of the medium, 4.4-5.2 wt% of glucose, 3.7-4.3 wt% of fructose, 1.3-1.7 wt% of magnesium sulfate, 2.0-2.4 wt% of phosphoric acid, 1.4-1.8 wt% of potassium hydroxide, and 0.5-0.9 wt% of the grain fermented broth. In addition, when the glutamic acid fermented broth is to be prepared, the main culture medium may comprise glucose, fructose, raw sugar, betaine, magnesium sulfate, potassium phosphate and phosphoric acid, and preferably, it may comprise, based on the total weight of the medium, 0.5-0.7 wt% of glucose, 0.9-1.1 wt% of fructose, 4.5-5.5 wt% of raw sugar, 0.005-0.015 wt% of betaine, 0.3-0.5 wt% of magnesium sulfate, 0.8-1.0 wt% of potassium phosphate and 0.2-0.4 wt% of phosphoric acid.

In addition, the medium may, if necessary, comprise small amounts of other components, for example, iron sulfate, manganese sulfate, copper sulfate, zinc sulfate, CAPA, NCA (nicotinamide), biotin, calcium chloride, thiamine, vitamin C, and the like. Typical examples of each of the media comprising these components are shown in Tables 5 to 8 and 9 to 12.

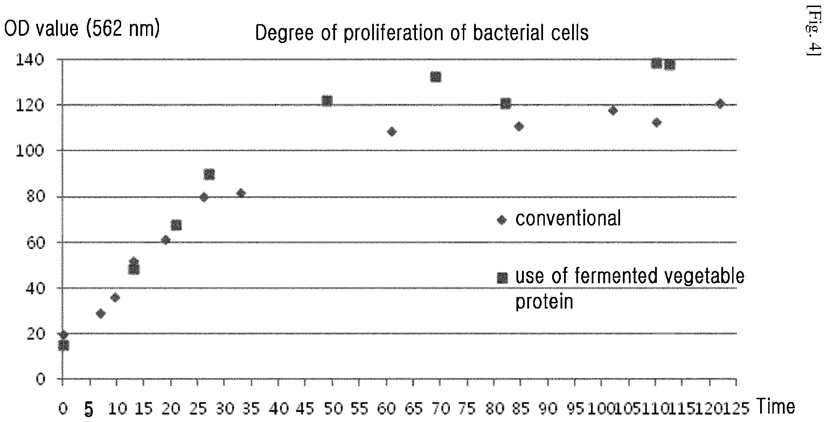

As a result of the fungal fermentation in step (a), the vegetable protein source is decomposed into amino acids and peptides, and inorganic ions, such as calcium, magnesium and phosphate ions, and vitamins are eluted from the protein source. The amino acids produced by the fungal fermentation include glutamine, cysteine, methionine, valine, leucine, isoleucine and the like, and may be used as a nitrogen source in the bacterial fermentation in step (b). Particularly, a high concentration of glutamine is an essential component for purine biosynthesis and can act as a major promoter for production of high contents of IMP and glutamic acid. In addition, the eluted inorganic ions and vitamins can be helpful in the growth of bacterial cells in the bacterial fermentation. In an example of the present invention, it was shown that, when the product obtained by the fungal fermentation was used as a nutrient source, the proliferation and growth of bacterial cells became faster (FIG. 4). This further demonstrates the advantage of the two-step process of the present invention.

The bacteria that are used in the present invention are *Corynebacterium* sp. microorganisms. Most preferably, *Corynebacterium ammoniagenes* may be used to prepare the IMP fermented broth, and *Corynebacterium glutamicum* may be used to prepare the glutamic acid fermented broth. In addition, as the bacteria, various bacteria, disclosed in previous patent documents and known to have the capability to produce IMP and glutamic acid, may be used. For example, the IMP fermented broth may be prepared the *Bacillus* sp. or *Escherichia* sp. bacteria disclosed in Korean Patent Laid-Open Publication No. 10-2007-000507 (corresponding to PCT International Patent Publication No. WO2005-095627), and the glutamic acid fermented broth may be prepared using the *Enterobacter* sp. or *Klebsiella* sp. disclosed in Korean Patent Laid-Open Publication No. 10-2000-0029174 (corresponding to US Patent No. 7247459), but are not limited thereto.

In an example of the present invention, to prepare an IMP fermented broth, *Corynebacterium ammoniagenes* CJIP009 (KCCM-10226) described in Korean Patent Registration No. 10-0397321 (corresponding to WO International Patent Publication No.WO2002-051984) was used, and to prepare a glutamic acid fermented broth, *Corynebacterium glutamicum* (*Brevibacterium lactofermentum*) CJ971010 (KFCC 11039) described in Korean Patent Registration No. 10-0264740 (published as KR 2000 0002408) was used.

The fermented broth obtained by bacterial fermentation in step (b) has a solid content of about 150 g/L. The present invention is characterized in that the IMP and/or glutamic acid fermented broth obtained by the bacterial fermentation is used in a natural flavor preparation process without adding any additional component thereto, the fermented broth should contain large amounts of IMP and glutamic acid that are the desired components.

Thus, the solid in the IMP fermented broth prepared by the first fermentation step for fungal fermentation and the second fermentation step for bacterial fermentation may preferably have an IMP content of 30% or more, and more preferably 50% or more, but is not limited thereto. Accordingly, the concentration of IMP in the IMP fermented broth may preferably be 50 g/L to 150 g/L, and more preferably 70 g/L to 130 g/L.

Further, because the product produced by the first fermentation step for fungal fermentation contains amino acids, the glutamic acid can be obtained at high concentration and high yield compared to those of IMP. The solid in the prepared glutamic acid fermented broth may preferably have a glutamic acid content of 50% or more, and more preferably 60% or more, but is not limited thereto. Thus, the concentration of glutamic acid in the glutamic acid fermented broth may preferably be 75 g/L to 150 g/ L, and more preferably 90 g/L to 130 g/L.

Due to high contents of IMP and glutamic acid in the fermented broth as described above, when the fermented broth is powdered by a process such as drying, it can be suitably added to food.

As used herein, the term "flavor" refers to a substance that is added to improve the flavor of food. The flavor can be classified according to its component into various flavors. Specific examples of the flavor include a neutral flavor, a beef flavor, a chicken flavor, a pork flavor, and a kokumi flavor. Each of the flavors can be prepared using the IMP fermented broth and glutamic acid fermented broth prepared by the two-step fermentation process of the present invention. Among them, the term "beef flavor" refers to a flavor that gives a roast beef taste/flavor even though it contains no animal raw material. The present inventors confirm that, when the beef flavor prepared by the method of the present invention is applied, the beef flavor and preference in a beef radish soup and a tomato meat source can be increased.

Because the fermented broth of the present invention is characterized in that it is used in a natural flavor preparation process without adding any additional components and without subjecting it to an additional chemical process such as purification, all medium components should be food grade materials in order for the fermented broth to be included directly in food, for example, processed food. Thus, all medium components that are added during fermentation are preferably food grade materials. In an example of the present invention, in order to use food grade materials as medium components, β-alanine was replaced with calcium panthothenate (CAPA), and nevertheless, it was shown that an IMP fermented broth having an IMP concentration of 70 g/L or more could be prepared. Thus, the medium for bacterial fermentation according to the present invention may preferably contain CAPA.

Each of the IMP fermented broth and the glutamic acid fermented broth can be prepared by the two-step fermentation process comprising steps (a) and (b), and the prepared fermented broth can finally be subjected to a third step for reaction and can be used in a process of preparing various flavors. Based on the IMP fermented broth and the glutamic acid fermented broth, various natural flavors, for example, neutral flavors, and flavors for beef, chicken, pork, kokumi and the like, can be prepared by using different raw materials, or slightly changing the medium composition, or controlling process conditions, including temperature, pressure and time, in the process of mixing the fermented broths, or a reaction or electrodialysis process. Among the prepared natural flavors, a flavor suitable for the purpose of each food may be added to the food to give the optimum taste.

Step (c) is a step of mixing the grain fermented broth and the IMP fermented broth obtained from the prior steps in order to prepare natural beef flavors. In addition, the step (c) may further comprise a step of mixing with the glutamic acid fermented broth.

In step (c), no additional component is added to the fermented broths, the fermented broths are not subjected to an additional chemical process, and the fermented broths are mixed with each other, and then reacted under suitable conditions, including temperature, pressure and time, according to the intended use of the desired flavor, thereby preparing natural beef flavors.

In step (c), natural beef flavors can be finally prepared by mixing the grain fermented broth and the IMP fermented broth through the preparation method of the present invention. Herein the mixing ratio of the grain fermented broth and the IMP fermented broth may preferably be 1:1 to 1:10, more preferably 1:2 to 1:8, and most preferably 1:4, but is not limited thereto. Moreover, the mixed fermented broth can further be mixed with the glutamic acid fermented broth.

After mixing each of the fermented broths as above, optimal beef flavors can be prepared by reacting at 80 °C to 120 °C, preferably 90 °C to 110 °C; and 0.8 to 1.5 bar, preferably 0.9 to 1.3 bar; for 0.5 to 24 hours, preferably 0.5 to 5 hours and more preferably 1 to 3 hours. Under above conditions, Maillard reaction can produce compounds such as 2,6-dimethylpyrazine, 2,3-dimethylpyrazine and dimethylpyrazine by high temperature, and due to these compounds, beef flavors can be formed.

Meanwhile, the fermented broths can be used after filtration process. Therefore, the inventive method may comprise a step of treating the fermented broths with activated carbon before step (c). In addition, the inventive method may comprise, after the step of treating the fermented broth with activated carbon, a step of centrifuging or filtering the fermented broth. After the fermented broth is treated with activated carbon, it may further be treated with diatomaceous earth as a filtration aid. Moreover, before the fermented broth is treated with activated carbon, it may be subjected to a pretreatment process of heating the fermented broth to induce cell lysis, in order to increase the yield of the filtration process that is subsequently carried out. The heating process may preferably be carried out at 70-90 °C, and the heating time may preferably be 15 minutes or longer, and more preferably 15-60 minutes.

The mixing step of the present invention may further comprise a step of concentrating the fermented broth and drying the concentrate to prepare powder. The step of preparing the fermented broth into powder may be performed before or after mixing the fermented broths, and can preferably be performed before mixing the fermented broths. The drying can preferably be achieved by spray drying or vacuum drying. The fermented broth can be finally prepared into powder or paste which is suitable for addition to food.

The natural beef flavor prepared by the preparation method of the present invention has a beef taste and flavor, and therefore may be added to a suitable food to maximize the taste of the food and may also be applied to animal food. For example, the natural beef flavor may be added to ham, sausage and the like to produce a beef flavor.

### Advantageous Effects of Invention

The natural beef flavors prepared according to the method of the present invention are prepared using natural raw materials, and thus are harmless and safe for use in the human body, and may be added to food to produce beef tastes and improve the flavor of the food.

### Brief Description of Drawings

FIG. 1 is a schematic view showing an overall process for preparing a natural flavor.
FIG. 2 shows a process of acid-hydrolyzing AMP in order to replace adenine with a natural food grade material according to the present invention.
FIG. 3 shows a metabolic pathway for deriving CAPA to replace β-alanine with a natural food grade material.
FIG. 4 shows the degree of proliferation of bacterial cells in the case in which a vegetable protein degraded by fungal fermentation according to the present invention was added as a nutrient source and in the case in which the vegetable protein was not added.

### Mode for the Invention

Hereinafter, the present invention will be described in further detail with reference to examples. However, it will be obvious to those skilled in the art that these examples are for illustrative purposes only and are not intended to limit the scope of the present invention.

### Example 1: Fungal fermentation of vegetable protein source

For primary fungal fermentation, a substrate including grain materials such as soybean, corn, rice or wheat was cultured using *Aspergillus* sp. microorganisms as a fungal strain at a temperature of 20∼35 °C for 24-72 hours, thereby preparing a fungal culture broth containing a high concentration of protease. Then, a vegetable protein source such as soybean, corn, rice or wheat was mixed with water to a high concentration of 25-35% and sterilized, and the above-prepared fungal culture broth containing a high concentration of protease was added to the sterilized vegetable protein source in a salt-free state to hydrolyze the vegetable protein source. Herein, the fungal culture broth was added in an amount of 10-100% based on the sterilized substrate solution, and the substrate was degraded at 40∼50 °C for 48-96 hours to prepare a grain protein hydrolysate.

Specifically, flask culture and expansion culture were performed using *Aspergillus sojae* CJCC_080124P (KCCM11026P) as described in Korean Patent Registration No. 10-1191010 (corresponding to PCT International Publication No. WO2011-046249), and then a fungal culture broth was prepared using defatted soybean, and wheat gluten as a substrate was hydrolyzed, thereby preparing a grain protein hydrolysate. More specifically, 200 ml of a primary culture was dispensed in a 1 L flask and sterilized, and 200 ml of fungal cells were inoculated in the flask at a density of 1.7×10¹⁰ fungal cells/400 ml and cultured at 30 °C and 100 rpm for 7 hours. Then, a secondary culture was added to a 250 L fermentor and sterilized, and 600 ml of the primary culture broth was inoculated therein and cultured at 30 °C and 70 rpm for 24 hours. Next, a main culture medium was added to a 5-ton preparatory tank and heated at 90 °C for 30 minutes, after it was transferred into a 8-ton fermentor, and 100 L of water and 2 L of a defoaming agent were added thereto. Then, 144 L of the secondary culture broth was inoculated in the main culture medium, and then cultured at 30 °C and 700 rpm for 48 hours, thereby preparing a fungal culture broth. Finally, a raw material for substrate hydrolysis was added to a 5-ton preparatory tank and heated at 55 °C for 1 hour, after which it was transferred into a 20-ton fermentor, and 100 L of water and 2 L of a defoaming agent were added thereto, followed by sterilization. Then, 5760 L of the fungal culture broth was inoculated in the substrate and cultured at 45 °C and 30 rpm for 96 hours, thereby preparing a grain protein hydrolysate. The compositions of the media used in the preparation of the grain protein hydrolysate are shown in Tables 1 to 4 below.

### Table 1

**[Table 1]**

| Primary culture for fungal fermentation | | | |
|---|---|---|---|
| Medium component | Unit | Amount | % |
| Glucose | g | 6 | 3 |
| KH₂PO₄ | g | 6 | 3 |
| Yeast extract | g | 6 | 3 |
| Water | ml | 182 | |

### Table 2

**[Table 2]**

| Secondary culture for fungal fermentation | | | |
|---|---|---|---|
| Medium component | Unit | Amount | % |
| Glucose | kg | 1.44 | 1 |
| KH₂PO₄ | kg | 1.44 | 1 |
| Yeast extract | kg | 1.44 | 1 |
| Water | kg | 138.6 | |

### Table 3

**[Table 3]**

| Main culture medium for fungal fermentation | | | |
|---|---|---|---|
| Medium component | Unit | Amount | % |
| Defatted soybean | kg | 103.68 | 1.9 |
| KH₂PO₄ | kg | 57.6 | 1 |
| Water | kg | 5352 | |

### Table 4

**[Table 4]**

| Raw material for substrate hydrolysis | | |
|---|---|---|
| Raw material | Unit | Amount |
| Wheat gluten | kg | 2650 |
| Water | L | 7500 |

The grain protein hydrolysate prepared as described above was filtered through a filter press to remove fungal cells, thereby preparing a grain fermented broth having a total nitrogen content of 2%(w/v) or more and a degree of hydrolysis of 50% or more. Meanwhile, the grain fermented broth was prepared as a medium for secondary bacterial fermentation.

### Example 2: Bacterial fermentation

To prepare an IMP fermented broth and a glutamic acid fermented broth using the grain fermented broth, a carbon source such as glucose or fructose, mineral salts such as Fe, Mg, Mn and Zn, and vitamins were added to the grain fermented broth prepared in Example 1, followed by sterilization, thereby preparing media for bacterial fermentation. The media for preparing an IMP fermented broth and a glutamic acid fermented broth were prepared, and for each of the fermented broths, media for flask culture (primary culture), expansion culture (secondary culture) and main culture were prepared.

### 2-1: Preparation of IMP fermented broth

Using *Corynebacterium ammoniagenes* CJIP009 (KCCM-10226) described in Korean Patent Registration No. 10-0397321 (corresponding to WO International Patent Publication No. WO2002-051984), an IMP fermented broth having an IMP concentration of 70g/L or more was prepared.

Specifically, 50 ml of a primary culture adjusted to a pH of 7.2 using NaOH was dispensed in a 500-ml flask and sterilized at 121 °C for 15 minutes, after the bacterial strain was inoculated into the medium and cultured at 32 °C and 200 rpm for 22-28 hours. Then, 2.1 L of a secondary culture was dispensed in a 5-L jar, sterilized and cooled, after which 300 ml of the primary culture broth was inoculated therein and cultured in 2 L of air at 32 °C and 900 rpm for 27-30 hours while adjusting the pH to 7.2. Next, 8.5 L of a main culture medium was dispensed in a 30-L jar, sterilized and cooled, after which 1500 ml of the secondary culture broth was inoculated therein and in 5 L of air at 32 °C and 400 rpm for 5-6 days while adjusting the pH to 7.2 and supplying an additional sugar comprising a mixture of glucose and fructose at any time. After supply of the final additional sugar, the culture was performed for 7 hours or longer so that the sugar could be completely consumed. The compositions of the media used in the preparation in the IMP fermented broth are shown in Tables 5 to 8 below.

### Table 5

**[Table 5]**

| Primary culture for preparation of IMP fermented broth | | | |
|---|---|---|---|
| Medium component | Unit | Amount | % |
| Glucose | g | 10 | 1 |
| Grain fermented broth | g | 35 | 3.5 |
| NaCl | g | 2.5 | 0.25 |
| Volume of medium | ml | 1000 | |

### Table 6

**[Table 6]**

| Secondary culture for preparation of IMP fermented broth | | | |
|---|---|---|---|
| Medium component | Unit | Amount | % |
| Glucose | g | 78.93 | 8 |
| Grain fermented broth | g | 21.43 | 2 |
| H₃PO₄ (85%) | g | 2.14 | 0.21 |
| KOH (85%) | g | 1.77 | 0.18 |
| MgSO₄7H₂O | g | 1.43 | 0.14 |
| (NH₄)₂SO₄ | g | 7.14 | 0.71 |
| L-cysteine HCl | mg | 28.6 | |
| CAPA | mg | 21.4 | |
| Biotin | mg | 0.09 | |
| Thiamine | mg | 7.1 | |
| FeSO₄7H₂O | mg | 28.6 | |
| MnSO₄7H₂O | mg | 14.3 | |
| ZnSO₄7H₂O | mg | 14.3 | |
| Volume of medium | ml | 1000 | |

### Table 7

**[Table 7]**

| Main medium for preparation of IMP fermented broth | | | |
|---|---|---|---|
| Medium component | Unit | Amount | % |
| H₃PO₄ (85%) | g | 22.21 | 2.2 |
| KOH (85%) | g | 15.86 | 1.6 |
| Grain fermented broth | g | 7 | 0.7 |
| CuSO₄5H₂O | mg | 12.2 | |
| FeSO₄7H₂O | mg | 36.5 | |
| ZnSO₄7H₂O | mg | 36.5 | |
| MnSO₄7H₂O | mg | 109.6 | |
| L-cysteine HCl | mg | 40.4 | |
| CAPA | mg | 34.7 | |
| NCA | mg | 34.9 | |
| Biotin | mg | 0.15 | |
| CaCl₂2H₂O | mg | 182.6 | |
| Thiamine | mg | 23.5 | |
| MgSO₄7H₂O | g | 14.61 | 1.5 |
| Glucose | g | 48.21 | 4.8 |
| Fructose | g | 40.22 | 4 |
| Volume of medium | ml | 1000 | |

### Table 8

**[Table 8]**

| Additional sugar for preparation of IMP fermented broth | | | |
|---|---|---|---|
| Medium component | Unit | Amount | % |
| Glucose | g | 358.97 | 35.9 |
| Fructose | g | 299.58 | 30 |
| NCA | mg | 772 | 0.08 |
| CAPA | mg | 936.6 | 0.09 |
| H₃PO₄ (85%) | g | 55.68 | 5.6 |
| NaOH (100%) | g | 13.5 | 1.4 |
| Volume of medium | ml | 1000 | |

### 2-2: Preparation of glutamic acid fermented broth

Using *Corynebacterium glutamicum* (*Brevibacterium lactofermentum*) CJ971010 (KFCC 11039) described in Korean Patent Registration No. 10-0264740, (published as KR 2000 0002408) a glutamic acid fermented broth having a glutamic acid concentration of 90 g/L or more was prepared.

Specifically, 30 ml of a primary culture was dispensed in a 250-ml flask and sterilized, and the bacterial strain was inoculated in the medium and cultured for 5-8 hours. Then, 1.4 L of a secondary culture was dispensed in a 5-L jar, sterilized and cooled, and then 20 ml of the primary culture broth was inoculated therein and cultured for 20-28 hours. Then, 9.2 L of a main culture medium was dispensed in a 30-L jar, sterilized and cooled, after which 800 ml of the secondary culture broth was inoculated therein and cultured for 36-45 hours while supplying an additional sugar at any time.

After supply of the final additional sugar, the culture was performed for 7 hours or longer so that the sugar could be completely consumed. The compositions of the media used in the preparation in the glutamic acid fermented broth are shown in Tables 9 to 12 below.

### Table 9

**[Table 9]**

| Primary culture for preparation of glutamic acid fermented broth | | | |
|---|---|---|---|
| Medium component | Unit | Amount | % |
| Raw sugar (98.5%) | g | 20.3 | 2 |
| Grain fermented broth | g | 13 | 1.3 |
| (NH₄)₂SO₄ | g | 13.72 | 1.4 |
| KH₂ PO₄ | g | 5.2 | 0.5 |
| K₂HPO₄ | g | 10.7 | 1 |
| MgSO₄7H₂O | g | 0.5 | 0.05 |
| d-biotin | mg | 1.8 | |
| Thiamine-HCl | mg | 9 | |
| CAPA | mg | 9 | |
| Niacinamide (nicotinamide) | mg | 60 | |
| Volume of medium | ml | 1000 | |

### Table 10

**[Table 10]**

| Secondary culture for preparation of glutamic acid fermented broth | | | |
|---|---|---|---|
| Medium component | Unit | Amount | % |
| Glucose | g | 81.79 | 8.2 |
| Fructose | g | 23.14 | 2.3 |
| H₃PO₄ (85%) | g | 2.73 | 0.27 |
| MgSO₄7H₂O | g | 1.03 | 0.1 |
| Betaine | g | 0.14 | 0.01 |
| DL-Meth | g | 0.15 | 0.01 |
| FeSO₄7H₂O | mg | 18.6 | |
| MnSO₄7H₂O | mg | 18.6 | |
| CuSO₄5H₂O | mg | 3.6 | |
| ZnSO₄7H₂O | mg | 3.6 | |
| Thiamine-HCl | mg | 7.1 | |
| Vitamin C (ascorbic acid) | mg | 7.1 | |
| Succinate | mg | 1.1 | |
| d-biotin | mg | 17.4 | |
| Vitamin B12 (cyanocobalamin) | mg | 0.057 | |
| Grain fermented broth | g | 42.86 | 4.3 |
| Volume of medium | ml | 1000 | |

### Table 11

**[Table 11]**

| Main culture medium for preparation of glutamic acid fermented broth | | | |
|---|---|---|---|
| Medium component | Unit | Amount | % |
| Glucose | g | 5.86 | 0.6 |
| Fructose | g | 10.48 | 1 |
| Raw sugar | g | 50 | 5 |
| Betaine | g | 0.97 | 0.1 |
| MgSO₄7H₂O | g | 3.69 | 0. 4 |
| K₂HPO₄ | g | 9 | 0. 9 |
| H₃PO₄ (85%) | g | 3.1 | 0.3 |
| FeSO₄7H₂O | mg | 22.9 | |
| MnSO₄7H₂O | mg | 22.9 | |
| CuSO₄5H₂O | mg | 2.3 | |
| ZnSO₄7H₂O | mg | 2.3 | |
| CAPA | mg | 2.9 | |
| NCA | mg | 2.9 | |
| Biotin | mg | 0.29 | |
| CaCl₂2H₂O | mg | 71.4 | |
| Thiamine-HCl | mg | 0.4 | |
| Vitamin C (ascorbic acid) | mg | 1.7 | |
| Volume of medium | ml | 1000 | |

### Table 12

**[Table 12]**

| Additional sugar for preparation of glutamic acid fermented broth | | | |
|---|---|---|---|
| Medium component | Unit | Amount | % |
| Glucose | g | 192.3 | 19.2 |
| Fructose | g | 54.33 | 5.4 |
| Raw sugar | g | 147.33 | 14.7 |
| Betaine | g | 0.67 | 0.07 |
| Grain fermented broth | g | 4 | 0.4 |
| CAPA | mg | 15.87 | |
| NCA | mg | 15.87 | |
| Volume of medium | ml | 1000 | |

In order to prepare the IMP fermented broth using the media for preparation of the IMP fermented broth and use the IMP fermented broth as a flavor for addition to food, several medium components should be replaced with food grade materials so as to satisfy the requirement of a food additive. Thus, adenine and β-alanine necessary for cell growth were replaced with food grade materials.

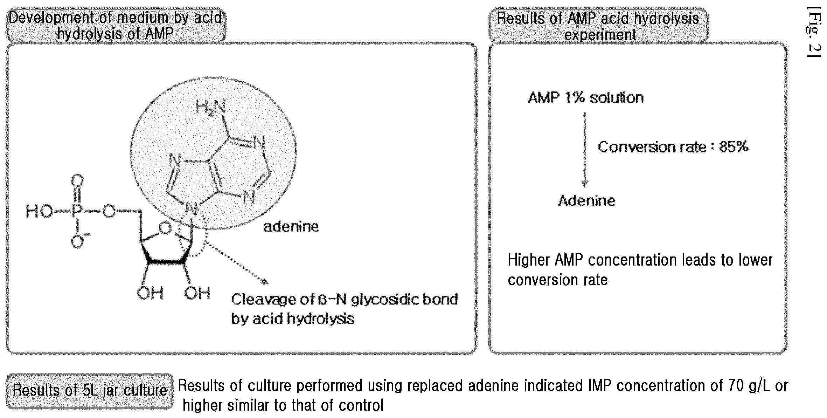

First, adenine was prepared by acid-hydrolyzing AMP to cleave a β-N-glycosidic bond with ribose to thereby liberate adenine. It was shown that, when culture was performed using the adenine prepared as described above, an IMP fermented broth having an IMP concentration of 70 g/L or higher could be prepared (FIG. 2).

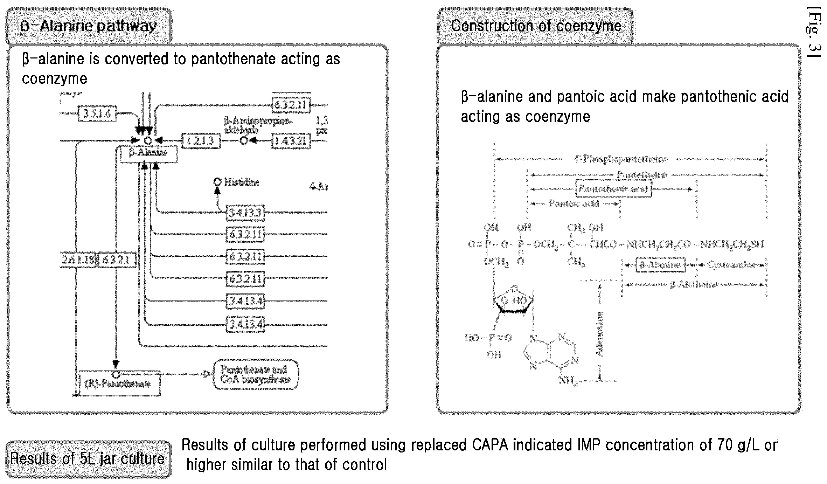

Then, as a result of examining the metabolic pathway of *Corynebacterium ammoniagenes,* it was expected that β-alanine would be replaced with calcium panthothenate (CAPA). Thus, culture was performed using CAPA in place of β-alanine, and as a result, it was shown that an IMP fermented broth having an IMP concentration of 70 g/ L or higher could be prepared (FIG. 3).

### Example 3: Examination of cell growth rate in case in which fungal fermentation product is used in bacterial fermentation

In order to confirm the advantage of the case in which the first fermentation step for fungal fermentation and the second fermentation step for bacterial fermentation are continuously performed, the growth rate and degree of proliferation of bacterial cells in the case in which the vegetable protein degraded by the first fungal fermentation step is added as a nutrient source and in the case in which the vegetable protein is not added were examined.

As a result, as can be seen in FIG. 4, in the case in which the vegetable protein degraded by the first fungal fermentation step was added as a nutrient source, the bacterial cells proliferated at a high rate and in a large amount compared to those in the case in which the vegetable protein was not added (FIG. 4).

### Example 4: Examination of the change in flavor characteristic of glutamic acid fermented broth according to the kind of carbon source

Carbon sources that may be used in glutamic acid fermentation include glucose fructose, cane molasses, raw sugar, etc. Although some other medium components vary depending on the kind of carbon source, the glutamic acid concentration of the resulting fermented broth does not significantly depend on the kind of carbon source. However, the flavor of the fermented broth significantly changes depending on the kind of carbon source, and to develop a natural flavor, the resulting fermented broth preferably has a cleaner flavor. Cane molasses is an excellent medium component in terms of microbial fermentation due to various inorganic ions contained therein, but has a shortcoming in that the value of use thereof as a natural flavor is low, because the color of the medium itself is too dark and a flavor caused by caramelization remains in the resulting culture broth.

Thus, raw sugar was used in place of cane molasses in the preparation of a glutamic acid fermented broth for preparing a natural flavor, and the resulting change in the flavor characteristic of the culture broth was examined. Herein, while cane molasses was replaced with raw sugar, major inorganic ions and vitamins contained in cane molasses were additionally added to the medium. As a result, it could be seen that, when cane molasses was replaced with raw sugar, off-flavor was removed, and thus a flavor characteristic suitable for preparing a natural flavor appeared (Table 13). In addition, the glutamic acid fermented broth prepared using raw sugar also contained a high concentration (96 g/L or more) of glutamic acid, which is a glutamic acid concentration suitable for preparing a natural flavor.

### Table 13

**[Table 13]**

| Change in flavor characteristic of culture broth as a function of ratio of cane molasses and raw sugar | | | |
|---|---|---|---|
| | Cane molasses (100%) | Cane molasses + raw sugar (5:5) | Raw sugar (100%) |
| Neutrality | - | + | ++ |
| Off-flavor | + | - | -- |

### Example 5: Preparation of beef flavor

Using the grain fermented broth prepared in Example 1 and IMP fermented broth prepared in Example 2, a beef flavor was prepared.

Specifically, in order to increase the yield of a filtration process to be performed subsequently, each of the fermented broths was heated at 70-90 °C for 15 minutes or more to induce cell lysis. This pretreatment process can increase the filtration yield to 85% or higher. Then, to remove off-flavor and off-odor from the glutamic acid fermented broth and the IMP fermented broth, activated carbon was added in an amount of 1-3% (w/v) based on the volume of the fermented broth, which was then treated with the activated carbon at 50 to 70 °C for 2 to 24 hours. After treatment with activated carbon, 1-3% (w/v) of diatomaceous earth as a filtration aid was added to the fermented broth, which was then filtered through a filter press. Then, the fermented broth filtered by the above process was used.

Meanwhile, the fermented broth obtained by the fungal fermentation and bacterial fermentation of the vegetable protein contains sulfur-containing amino acids such as cysteine and methionine, organic acids, sugars and the like, derived from the vegetable protein and produced by the metabolic pathway of bacteria.

The grain fermented broth and the IMP fermented broth were mixed with each other at a ratio of 1:4 and subjected to a Maillard reaction at 80∼120 °C and 0.8-1.5 bar for 0.5-24 hours. Herein, in 10% of the IMP produced by bacterial fermentation, the bond between the phosphate group and pentose is cleaved by high temperature to produce pentose, and the produced pentose can be used as a precursor for the Maillard reaction, and thus a beef flavor can be prepared even when adding additional sugar. The Maillard reaction produced amadori rearrangement compounds, 2,6-dimethylpyrazine, 2,3-dimethylpyrazine and dimethylpyrazine, and due to these compounds, a beef flavor was formed.

In another reaction route, after completion of the bacterial fermentation, ammonia was not supplied in the late stage of the reaction to adjust the pH to 5-7, and 3,4-dideoxy glycosulos-3-ene was produced by the high temperature/high pressure reaction of components in the culture broth, followed by the production of furan compounds, thereby forming a beef flavor.

The reaction solution prepared by the above-described reaction was spray-dried or vacuum-dried, thereby preparing a beef flavor. Specifically, refined salt and dextrin were added to the reaction solution to a solid content of 35-50%, and the mixture was spray-dried or vacuum-dried to form powder or was concentrated to a solid content of 70% to form a paste. The beef flavor can show an effect like adding beef by the reaction of the microbial fermented broth, even though it contains no animal raw material.

### Example 6: Evaluation of sensory attributes resulting from application of beef flavor

In order to examine whether the application of the beef flavor of the present invention increases the flavor of beef compared to the use of a conventional seasoning having a beef flavor, the evaluation of sensory attributes was performed.

### 6-1: Beef radish soup

Beef radish soup to which a conventional beef flavor was added was prepared, and the beef flavor of the present invention was additionally added thereto. The sensory attributes of the beef flavor before and after addition of the beef flavor of the present invention were evaluated. The sensory evaluation was performed by 84 housewives who used seasonings.

Sensory attributes such as preference and intensity were evaluated on a 5-point scale. As a result, as shown in Table 14 below, in the group to which the beef flavor was applied, the preference of the overall taste was higher by 0.2 points than that before application of the beef flavor, and particularly, the intensity and preference of beef flavor after application of the beef flavor were significantly higher than those before application of the beef flavor.

### Table 14

**[Table 14]**

| Attributes | Conventional | After application of beef flavor | Difference (between after and before application) |
|---|---|---|---|
| Preference of overall taste | 3.4 | 3.6 | 0.2 |
| Preference of appearance | 3.5 | 3.7 | 0.2 |
| Preference of beef flavor | 3.3 | 3.5 | 0.2 |
| Preference of deep taste | 3.0 | 3.4 | 0.4 |
| Intensity of beef flavor | 3.0 | 3.6 | 0.6 |
| Umami intensity | 3.2 | 3.5 | 0.3 |
| Feeling of deep taste | 2.9 | 3.3 | 0.4 |

### 6-2: Tomato meat source

To a tomato meat source comprising the same amounts of tomato and meat, the powdered beef flavor was added, and the sensory attributes of the tomato meat source before and after addition of the beef flavor were evaluated by 80 housewives. Sensory attributes such as preference and intensity were evaluated on a 5-point scale.

As a result, as shown in Table 15 below, in the group to which the beef flavor was applied, the preference of the overall taste was higher by 0.2 points than that before application of the beef flavor, and the intensity and preference of meat flavor after application of the beef flavor were significantly higher than those before application of the beef flavor. Such results suggest that, even when the beef flavor is applied to a meat flavor-based product, it can increase the meat flavor of the product to increase the overall taste quality of the product.

### Table 15

**[Table 15]**

| Attributes | Conventional | After application of beef flavor | Difference (between after and before application) |
|---|---|---|---|
| Preference of overall taste | 3.6 | 3,8 | 0.2 |
| Preference of umami taste | 3.5 | 3.7 | 0.2 |
| Intensity of umami taste | 3.7 | 3.7 | 0 |
| Preference of tomato flavor | 3.8 | 3.8 | 0 |
| Intensity of tomato flavor | 3.5 | 3.7 | 0.2 |
| Preference of meat flavor of product | 3.7 | 3.9 | 0.2 |
| Intensity of meat flavor of product | 3.5 | 3.8 | 0.3 |

## Claims

1. A method for preparing a natural beef flavor, comprising:
(a) fermenting a vegetable protein source with *Aspergillus* sp. microorganism to obtain a grain fermented broth;
(b) fermenting a part of the grain fermented broth with *Corynebacterium* sp. or *Brevibacterium* sp. microorganism to prepare an inosine-5'-monophosphate (IMP) fermented broth; and
(c) mixing another part of the grain fermented broth of step (a) and the IMP fermented broth of step (b), and reacting the mixture at 80 to 120°C at 0.8 to 1.5 bar for 0.5 to 24 hours.

2. The method of claim 1, wherein step (b) further comprises fermenting another part of the grain fermented broth with *Corynebacterium* sp. or *Brevibacterium* sp. microorganism to prepare a glutamic acid fermented broth, and step (c) further comprises mixing the glutamic acid fermented broth and the mixture obtained from step (c) of claim 1.

3. The method of claim 1, wherein the natural flavor is prepared using only the grain fermented broth and IMP fermented broth without adding any additional component in step c).

4. The method of claim 2, wherein the natural flavor is prepared using only the grain fermented broth, IMP fermented broth and glutamic acid fermented broth without adding any additional component in step c).

5. The method of claim 1, wherein the natural flavor is prepared using the grain fermented broth and IMP fermented broth without subjecting them to any additional chemical process in step c).

6. The method of claim 2, wherein the natural flavor is prepared using the grain fermented broth, IMP fermented broth and glutamic acid fermented broth without subjecting them to any additional chemical process in step c).

7. The method of claim 1 or 2, wherein the vegetable protein source is selected from the group consisting of soybean, corn, rice, wheat, and wheat gluten.

8. The method of claim 1 or 2, wherein the *Aspergillus* sp. microorganism is *Aspergillus sojae.*

9. The method of claim 1, wherein *Corynebacterium* sp. microorganism is *Corynebacterium ammoniagenes* or *Corynebacterium glutamicum.*

10. The method of claim 1 or 2, wherein the grain fermented broth and the IMP fermented broth are mixed at a ratio of 1:1 to 1:10.

11. The method of claim 1, wherein the *Brevibacterium* sp. microorganism is *Brevibacterium lactofermentum.*

12. The method of claim 1 or 2, wherein a medium composition for bacterial fermentation for preparing the IMP fermented broth or the glutamic acid fermented broth comprises a food grade material, and optionally comprises calcium panthothenate (CAPA).

13. The method of claim 1, wherein a medium for bacterial fermentation for preparing the IMP fermented broth comprises glucose, fructose, magnesium sulfate, phosphoric acid, potassium hydroxide and a grain fermented broth.

14. The method of claim 2, wherein a medium for bacterial fermentation for preparing the glutamic acid fermented broth comprises glucose, fructose, raw sugar, betaine, magnesium sulfate, potassium phosphate and phosphoric acid.

15. The method of claim 2, wherein a carbon source in a medium for bacterial fermentation for preparing the glutamic acid fermented broth is raw sugar.

16. The method of claim 1, wherein the concentration of IMP in the prepared IMP fermented broth of step (b) is 50 g/L to 150 g/L, or the content of IMP in solids in the prepared IMP fermented broth of step (b) is 30 wt% or more.

17. The method of claim 2, wherein the concentration of glutamic acid in the prepared glutamic acid fermented broth of step (b) is 75 g/L to 150 g/L, or the content of glutamic acid in solids in the prepared glutamic acid fermented broth of step (b) is 50 wt% or more.

18. The method of claim 1, further comprising treating the IMP fermented broth with activated carbon before step (c); and optionally further comprising:
(i) after treating the fermented broth with activated carbon, centrifuging or filtering the treated fermented broth, or
(ii) before treating the fermented broth with activated carbon, heating the fermented broth at a temperature of 70 to 90 °C for 15 to 60 minutes to induce cell lysis.

19. The method of claim 2, further comprising treating the IMP fermented broth and the glutamic acid fermented broth with activated carbon before step (c); and optionally further comprising:
(i) after treating the fermented broth with activated carbon, centrifuging or filtering the treated fermented broth, or
(ii) before treating the fermented broth with activated carbon, heating the fermented broth at a temperature of 70 to 90 °C for 15 to 60 minutes to induce cell lysis.

20. The method of claim 1, further comprising concentrating the mixture of grain fermented broth and IMP fermented broth; and drying the concentrate to prepare powder in step (c).

21. The method of claim 2, further comprising concentrating the mixture of grain fermented broth, IMP fermented broth, and glutamic acid fermented broth; and drying the concentrate to prepare powder in step (c).

## Patentansprüche

1. Verfahren zur Herstellung eines natürlichen Rindergeschmacks, umfassend:
(a) Fermentieren einer pflanzlichen Proteinquelle mit dem Mikroorganismus *Aspergillus sp.,* um eine Brühe aus fermentiertem Getreide zu erhalten;
(b) Fermentieren eines Teils der Brühe aus fermentiertem Getreide mit dem Mikroorganismus *Corynebacterium sp.* oder *Brevibacterium sp.,* um eine Brühe aus fermentiertem Inosin-5 '-monophosphat (IMP) herzustellen; und
(c) Mischen eines weiteren Teils der Brühe aus fermentiertem Getreide aus Schritt (a) und der Brühe aus fermentiertem IMP aus Schritt (b) und Reagieren der Mischung bei 80 bis 120 °C bei 0,8 bis 1,5 bar während 0,5 bis 24 Stunden.

2. Verfahren nach Anspruch 1, wobei Schritt (b) weiter das Fermentieren eines weiteren Teils der Brühe aus fermentiertem Getreide mit dem Mikroorganismus *Corynebacterium sp.* oder *Brevibacterium sp.* umfasst, um eine Brühe aus fermentierter Glutaminsäure herzustellen, und der Schritt (c) weiter das Mischen der Brühe aus fermentierter Glutaminsäure und der Mischung, erhalten aus Schritt (c) von Anspruch 1, umfasst.

3. Verfahren nach Anspruch 1, wobei der natürliche Geschmack hergestellt wird unter Verwendung nur der Brühe aus fermentiertem Getreide und der Brühe aus fermentiertem IMP ohne Zugabe einer zusätzlichen Komponente in Schritt c).

4. Verfahren nach Anspruch 2, wobei der natürliche Geschmack hergestellt wird unter Verwendung nur der Brühe aus fermentiertem Getreide, der Brühe aus fermentiertem IMP und der Brühe aus fermentierter Glutaminsäure, ohne Zugabe einer zusätzlichen Komponente in Schritt c).

5. Verfahren nach Anspruch 1, wobei der natürliche Geschmack hergestellt wird unter Verwendung der Brühe aus fermentiertem Getreide und der Brühe aus fermentiertem IMP, ohne sie einem zusätzlichen chemischen Prozess in Schritt c) zu unterziehen.

6. Verfahren nach Anspruch 2, wobei der natürliche Geschmack hergestellt wird unter Verwendung der Brühe aus fermentiertem Getreide, der Brühe aus fermentiertem IMP und der Brühe aus fermentierter Glutaminsäure, ohne sie einem zusätzlichen chemischen Prozess in Schritt c) zu unterziehen.

7. Verfahren nach Anspruch 1 oder 2, wobei die pflanzliche Proteinquelle ausgewählt ist aus der Gruppe, bestehend aus Sojabohnen, Mais, Reis, Weizen und Weizengluten.

8. Verfahren nach Anspruch 1 oder 2, wobei der Mikroorganismus *Aspergillus sp. Aspergillus sojae* ist.

9. Verfahren nach Anspruch 1, wobei der Mikroorganismus *Corynebacterium sp. Corynebacterium ammoniagenes* oder *Corynebacterium glutamicum* ist.

10. Verfahren nach Anspruch 1 oder 2, wobei die Brühe aus fermentiertem Getreide und die Brühe aus fermentiertem IMP in einem Verhältnis von 1:1 bis 1:10 gemischt werden.

11. Verfahren nach Anspruch 1, wobei der Mikroorganismus *Brevibacterium sp. Brevibacterium lactofermentum* ist.

12. Verfahren nach Anspruch 1 oder 2, wobei eine mittlere Zusammensetzung für bakterielle Fermentierung zur Herstellung der Brühe aus fermentiertem IMP oder der Brühe aus fermentierter Glutaminsäure ein Nahrungsmittelmaterial umfasst und optional Calciumpanthothenat (CAPA) umfasst.

13. Verfahren nach Anspruch 1, wobei ein Mittel zur bakteriellen Fermentierung zur Herstellung der Brühe aus fermentiertem IMP Glucose, Fructose, Magnesiumsulfat, Phosphorsäure, Kaliumhydroxid und eine Brühe aus fermentiertem Getreide umfasst.

14. Verfahren nach Anspruch 2, wobei ein Mittel zur bakteriellen Fermentierung zur Herstellung der Brühe aus fermentierter Glutaminsäure Glucose, Fructose, Rohzucker, Betain, Magnesiumsulfat, Kaliumphosphat und Phosphorsäure umfasst.

15. Verfahren nach Anspruch 2, wobei eine Kohlenstoffquelle in einem Mittel zur bakteriellen Fermentierung zur Herstellung der Brühe aus fermentierter Glutaminsäure Rohzucker ist.

16. Verfahren nach Anspruch 1, wobei die Konzentration von IMP in der hergestellten Brühe aus fermentiertem IMP von Schritt (b) 50 g/L bis 150 g/L ist, oder der Gehalt von IMP in Feststoffen und der hergestellten Brühe aus fermentiertem IMP aus Schritt (b) 30 Gew.% oder mehr ist.

17. Verfahren nach Anspruch 2, wobei die Konzentration von Glutaminsäure in der hergestellten Brühe aus fermentierter Glutaminsäure von Schritt (b) 75 g/L bis 150 g/L ist, oder der Gehalt von Glutaminsäure in Feststoffen in der hergestellten Brühe aus fermentierter Glutaminsäure von Schritt (b) 50 Gew.% oder mehr ist.

18. Verfahren nach Anspruch 1, weiter umfassend Behandeln der Brühe aus fermentiertem IMP mit Aktivkohle vor Schritt (c); und optional weiter umfassend:
(i) nach der Behandlung der fermentierten Brühe mit Aktivkohle Zentrifugieren oder Filtern der behandelten fermentierten Brühe, oder
(ii) vor der Behandlung der fermentierten Brühe mit Aktivkohle Erhitzen der fermentierten Brühe auf eine Temperatur von 70 bis 90 °C während 15 bis 60 Minuten, um eine Zelllyse zu induzieren.

19. Verfahren nach Anspruch 2, weiter umfassend Behandeln der Brühe aus fermentiertem IMP und der Brühe aus fermentierter Glutaminsäure mit Aktivkohle vor Schritt (c); und optional weiter umfassend:
(i) nach der Behandlung der fermentierten Brühe mit Aktivkohle Zentrifugieren oder Filtern der behandelten fermentierten Brühe, oder
(ii) vor der Behandlung der fermentierten Brühe mit Aktivkohle Erhitzen der fermentierten Brühe auf eine Temperatur von 70 bis 90 °C während 15 bis 60 Minuten, um eine Zelllyse zu induzieren.

20. Verfahren nach Anspruch 1, weiter umfassend Konzentrieren der Mischung aus Brühe aus fermentiertem Getreide und der Brühe aus fermentiertem IMP; und Trocknen des Konzentrats, um Pulver in Schritt (c) herzustellen.

21. Verfahren nach Anspruch 2, weiter umfassend Konzentrieren der Mischung aus Brühe aus fermentiertem Getreide, der Brühe aus fermentiertem IMP um der Brühe aus fermentierter Glutaminsäure; und Trocknen des Konzentrats, um Pulver in Schritt (c) herzustellen.

## Revendications

1. Méthode de préparation d'un arôme naturel de boeuf, comprenant :
(a) la fermentation d'une source de protéine végétale avec un microorganisme de l'espèce *Aspergillus* pour obtenir un bouillon fermenté de grains ;
(b) la fermentation d'une partie du bouillon fermenté de grains avec un microorganisme de l'espèce *Corynebacterium* ou de l'espèce *Brevibacterium* pour préparer un bouillon fermenté d'inosine-5'-monophosphate (IMP) ; et
(c) le mélange d'une autre partie du bouillon fermenté de grains de l'étape (a) et du bouillon fermenté d'IMP de l'étape (b), et la réaction du mélange à 80 à 120 °C à 0,8 bar à 1,5 bar pendant 0,5 à 24 heures.

2. Méthode selon la revendication 1, dans laquelle l'étape (b) comprend en outre la fermentation d'une autre partie du bouillon fermenté de grains avec un microorganisme de l'espèce *Corynebacterium* ou de l'espèce *Brevibacterium* pour préparer un bouillon fermenté d'acide glutamique, et l'étape (c) comprend en outre le mélange du bouillon fermenté d'acide glutamique et du mélange obtenu à l'étape (c) selon la revendication 1.

3. Méthode selon la revendication 1, dans laquelle l'arôme naturel est préparé en utilisant uniquement le bouillon fermenté de grains et le bouillon fermenté d'IMP sans ajouter aucun composant additionnel à l'étape c).

4. Méthode selon la revendication 2, dans laquelle l'arôme naturel est préparé en utilisant uniquement le bouillon fermenté de grains, le bouillon fermenté d'IMP et le bouillon fermenté d'acide glutamique sans ajouter aucun composant additionnel à l'étape c).

5. Méthode selon la revendication 1, dans laquelle l'arôme naturel est préparé en utilisant le bouillon fermenté de grains et le bouillon fermenté d'IMP sans les soumettre à un procédé chimique additionnel à l'étape c) .

6. Méthode selon la revendication 2, dans laquelle l'arôme naturel est préparé en utilisant le bouillon fermenté de grains, le bouillon fermenté d'IMP et le bouillon fermenté d'acide glutamique sans les soumettre à un procédé chimique additionnel à l'étape c).

7. Méthode selon la revendication 1 ou 2, dans laquelle la source de protéine végétale est choisie dans le groupe constitué du soja, du maïs, du riz, du blé et du gluten de blé.

8. Méthode selon la revendication 1 ou 2, dans laquelle le microorganisme de l'espèce *Aspergillus* est *Aspergillus sojae.*

9. Méthode selon la revendication 1, dans laquelle le microorganisme de l'espèce *Corynebacterium* est *Corynebacterium ammoniagenes* ou *Corynebacterium glutamicum.*

10. Méthode selon la revendication 1 ou 2, dans laquelle le bouillon fermenté de grains et le bouillon fermenté d'IMP sont mélangés dans un rapport de 1/1 à 1/10.

11. Méthode selon la revendication 1, dans laquelle le microorganisme de l'espèce *Brevibacterium* est *Brevibacterium lactofermentum.*

12. Méthode selon la revendication 1 ou 2, dans laquelle une composition de milieu pour fermentation bactérienne pour préparer le bouillon fermenté d'IMP ou le bouillon fermenté d'acide glutamique comprend une matière de qualité alimentaire, et comprend facultativement du panthothénate de calcium (CAPA).

13. Méthode selon la revendication 1, dans laquelle un milieu pour fermentation bactérienne pour préparer le bouillon fermenté d'IMP comprend du glucose, du fructose, du sulfate de magnésium, de l'acide phosphorique, de l'hydroxyde de potassium et un bouillon fermenté de grains.

14. Méthode selon la revendication 2, dans laquelle un milieu pour fermentation bactérienne pour préparer le bouillon fermenté d'acide glutamique comprend du glucose, du fructose, un sucre brut, de la bétaïne, du sulfate de magnésium, du phosphate de potassium et de l'acide phosphorique.

15. Méthode selon la revendication 2, dans laquelle une source de carbone dans un milieu pour fermentation bactérienne pour préparer le bouillon fermenté d'acide glutamique est un sucre brut.

16. Méthode selon la revendication 1, dans laquelle la concentration d'IMP dans le bouillon fermenté d'IMP préparé de l'étape (b) est de 50 g/l à 150 g/l, ou la teneur en IMP des solides dans le bouillon fermenté d'IMP préparé de l'étape (b) est de 30 % en poids ou plus.

17. Méthode selon la revendication 2, dans laquelle la concentration d'acide glutamique dans le bouillon fermenté d'acide glutamique préparé de l'étape (b) est de 75 g/l à 150 g/l, ou la teneur en acide glutamique des solides dans le bouillon fermenté d'acide glutamique préparé de l'étape (b) est de 50 % en poids ou plus.

18. Méthode selon la revendication 1, comprenant en outre le traitement du bouillon fermenté d'IMP avec du charbon actif avant l'étape (c) ; et facultativement comprenant en outre :
(i) après le traitement du bouillon fermenté avec du charbon actif, la centrifugation ou la filtration du bouillon fermenté traité, ou
(ii) avant le traitement du bouillon fermenté avec du charbon actif, le chauffage du bouillon fermenté à une température de 70 à 90 °C pendant 15 à 60 minutes pour induire une lyse cellulaire.

19. Méthode selon la revendication 2, comprenant en outre le traitement du bouillon fermenté d'IMP et du bouillon fermenté d'acide glutamique avec du charbon actif avant l'étape (c) ; et facultativement comprenant en outre :
(i) après le traitement du bouillon fermenté avec du charbon actif, la centrifugation ou la filtration du bouillon fermenté traité, ou
(ii) avant le traitement du bouillon fermenté avec du charbon actif, le chauffage du bouillon fermenté à une température de 70 à 90 °C pendant 15 à 60 minutes pour induire une lyse cellulaire.

20. Méthode selon la revendication 1, comprenant en outre la concentration du mélange de bouillon fermenté de grains et de bouillon fermenté d'IMP ; et le séchage du concentré pour préparer une poudre à l'étape (c).

21. Méthode selon la revendication 2, comprenant en outre la concentration du mélange de bouillon fermenté de grains, de bouillon fermenté d'IMP et de bouillon fermenté d'acide glutamique ; et le séchage du concentré pour préparer une poudre à l'étape (c).
